# EUROPÄISCHE PATENTSCHRIFT

(11) **EP 2 493 337 B1**
(45) Veröffentlichungstag und Bekanntmachung des Hinweises auf die Patenterteilung: **25.05.2016**
(21) Anmeldenummer: 10763374.5
(22) Anmeldetag: 12.10.2010
(51) Int. Cl.: A61K 8/97, B01D 11/02

(54) **VERFAHREN ZUR HERSTELLUNG EINES CARNOSOLSÄUREREICHEN PFLANZENEXTRAKTS**
METHOD FOR PRODUCING A CARNOSIC ACID-RICH PLANT EXTRACT
PROCÉDÉ DE PRODUCTION D'UN EXTRAIT VÉGÉTAL RICHE EN ACIDE CARNOSIQUE

(30) Priorität: 26.10.2009 DE 102009045994
(43) Veröffentlichungstag der Anmeldung: 05.09.2012
(73) Patentinhaber: Evonik Degussa GmbH, 45128 Essen (DE)
(72) Erfinder: WIESMUELLER, Johann, 84518 Garching (DE); MICHLBAUER, Franz, 84558 Kirchweidach (DE); KAHLEYSS, Ralf, 83368 St. Georgen (DE); HAUSNER, Helmut, 83308 Trostberg (DE)
(86) Internationale Anmeldenummer: PCT/EP2010/065268
(87) Internationale Veröffentlichungsnummer: WO 2011/054631

(56) Entgegenhaltungen:
- EP-A1- 0 454 097
- DE-A1- 4 306 303

## Beschreibung

Die Erfindung richtet sich auf ein Verfahren zur Herstellung eines carnosolsäurereichen Pflanzenextrakts durch Extraktion eines carnosolsäurehaltigen Pflanzenmaterials mit überkritischem CO₂.

Carnosolsäure ist ein pflanzlicher Inhaltsstoff, der in Lebensmitteln oder Kosmetika als natürliches Antioxidationsmittel an Stelle der bisher häufig verwendeten Antioxidationsmittel Butylhydroxyanisol (BHA) und Butylhydroxytoluol (BHT) verwendet werden kann. Für eine solche Anwendung werden carnosolsäurereiche Pflanzenextrakte benötigt, die eine geringe Eigenfarbe und einen geringen Gehalt an geruchs- und geschmacksgebenden Stoffen aufweisen und die möglichst kein Lösungsmittel enthalten sollen.

EP 454 097 offenbart Verfahren zur Herstellung von carnosolsäurereichen Extrakten mit einem geringen Gehalt an etherischen Ölen aus Rosmarin oder Salbei. Dazu wird entweder eine zweistufige Extraktion mit überkritischem CO₂ bei zuerst 300 bis 350 bar und 35 bis 40 °C und anschließend 500 bar und 40 °C durchgeführt oder es wird bei 500 bar und 40 °C mit überkritischem CO₂ extrahiert und aus dem erhaltenen CO₂-Extrakt in zwei Druckstufen bei zuerst 115 bis 120 bar und 75 bis 85 °C und anschließend 33 bis 35 bar und 10 bis 17 °C fraktioniert jeweils ein Extrakt abgeschieden. Das Verfahren liefert Extrakte mit einem Gehalt an Carnosolsäure von mehr als 25 Gew.-%, die allerdings noch grünlich braun gefärbt sind. Das Verfahren hat den Nachteil, dass bei unterschiedlichen Druckstufen gearbeitet werden muss und dass sich bei einer Abscheidung in zwei Druckstufen der in der ersten Druckstufe erhaltene viskose Extrakt nur schlecht von der noch überkritischen CO₂-reichen Phase abtrennen lässt.

DE 43 06 303 offenbart Verfahren zur Gewinnung von pflanzlichen Ölen durch Extraktion überkritischem CO₂, bei denen der CO₂-Extrakt unter überkritischen Bedingungen über eine Schüttung von Bleicherde geleitet wird, um Farbstoffe zu entfernen.

Gegenstand der Erfindung ist ein Verfahren zur Herstellung eines carnosolsäurereichen Pflanzenextrakts, das eine Extraktion eines getrockneten und zerkleinerten carnosolsäurehaltigen Pflanzenmaterials mit überkritischem CO₂ bei einem Druck von mindestens 200 bar und einer Temperatur von höchstens 100 °C umfasst. Bei dieser Extraktion wird in einem ersten Schritt überkritisches CO₂ in einer Menge von 10 bis 50 kg CO₂ je kg Pflanzenmaterial durch das Pflanzenmaterial geleitet unter Erhalt eines ersten CO₂ -Extrakts, in einem zweiten Schritt wird weiteres überkritisches CO₂ durch das Pflanzenmaterial geleitet unter Erhalt eines zweiten CO₂-Extrakts und aus dem zweiten CO₂-Extrakt wird durch Absenken des Drucks ein carnosolsäurereicher Pflanzenextrakt abgeschieden.

Das erfindungsgemäße Verfahren liefert einen Pflanzenextrakt mit einem hohen Gehalt an Carnosolsäure und weist nicht die Nachteile des aus EP 454 097 bekannten Verfahrens auf.

In dem erfindungsgemäßen Verfahren wird ein getrocknetes und zerkleinertes carnosolsäurehaltiges Pflanzenmaterial extrahiert. Vorzugsweise wird ein Pflanzenmaterial eingesetzt, das auf einen Wassergehalt von weniger als 10 Gew.-% getrocknet ist. Für die Trocknung des Pflanzenmaterials können alle aus dem Stand der Technik bekannten Trocknungsverfahren eingesetzt werden. Vorzugsweise erfolgt die Trocknung bei Temperaturen von weniger als 100 °C, besonders bevorzugt bei weniger als 85 °C. Das Pflanzenmaterial wird vorzugsweise unzerkleinert und/oder unter Ausschluss von Luftsauerstoff getrocknet, um eine Reaktion von darin enthaltener Carnosolsäure mit Luftsauerstoff während der Trocknung zu vermeiden. Das Pflanzenmaterial wird für die Extraktion zerkleinert eingesetzt. Vorzugsweise wird das Pflanzenmaterial auf eine gewichtsbezogene mittlere Partikelgröße von weniger als 1,5 mm zerkleinert. Für die Zerkleinerung des Pflanzenmaterials können alle aus dem Stand der Technik bekannten Zerkleinerungsverfahren eingesetzt werden. Vorzugsweise erfolgt die Zerkleinerung bei Temperaturen von weniger als 40 °C. Das Pflanzenmaterial wird vorzugsweise unter Ausschluss von Luftsauerstoff zerkleinert, um eine Reaktion von darin enthaltener Carnosolsäure mit Luftsauerstoff während des Zerkleinerns zu vermeiden.

Als carnosolsäurehaltiges Pflanzenmaterial eignen sich Pflanzenteile von Pflanzen aus der Familie der Labiatae, vorzugsweise Pflanzenteile von Pflanzen der Gattungen Rosmarinum und Salvia. Besonders bevorzugt enthält das Pflanzenmaterial Blätter von Rosmarin (Rosmarinum officinalis) oder Salbei (Salvia officinalis).

Das getrocknete und zerkleinerte carnosolsäurehaltige Pflanzenmaterial wird in dem erfindungsgemäßen Verfahren mit überkritischem CO₂ bei einem Druck von mindestens 200 bar und einer Temperatur von höchstens 100 °C extrahiert. Die Temperatur muss bei der Extraktion mehr als 31 °C betragen, damit das CO₂ im überkritischen Zustand vorliegt. Vorzugsweise wird bei einem Druck von 280 bis 1000 bar und besonders bevorzugt bei einem Druck von 280 bis 420 bar extrahiert.

Geeignete Autoklaven für eine Extraktion mit überkritischem CO₂ sind dem Fachmann aus dem Stand der Technik bekannt. Vorzugsweise wird zur Extraktion überkritisches CO₂ durch eine Schicht aus carnosolsäurehaltigem Pflanzenmaterial geleitet. Das überkritische CO₂ kann dabei sowohl in Aufwärtsströmung als auch in Abwärtsströmung durch die Schicht aus Pflanzenmaterial geleitet werden. Das CO₂ kann dabei sowohl in reiner Form als auch gemischt mit bis zu 10 Gew.-% eines aus dem Stand der Technik bekannten Entrainers eingesetzt werden. Als Entrainer werden vorzugsweise aliphatische Alkohole mit bis zu 4 Kohlenstoffatomen, Alkane mit bis zu 6 Kohlenstoffatomen und aliphatische Ketone mit bis zu 5 Kohlenstoffatomen verwendet. Vorzugsweise wird allerdings ohne Entrainer extrahiert.

Bei dem erfindungsgemäßen Verfahren wird in einem ersten Schritt das überkritische CO₂ in einer Menge von 10 bis 50 kg CO₂ je kg Pflanzenmaterial durch das Pflanzenmaterial geleitet unter Erhalt eines ersten CO₂-Extrakts. In einem zweiten Schritt wird dann weiteres überkritisches CO₂ durch das Pflanzenmaterial geleitet unter Erhalt eines zweiten CO₂-Extrakts. Vorzugsweise werden dabei weitere 80 bis 250 kg CO₂ je kg Pflanzenmaterial durch das Pflanzenmaterial geleitet. Erfindungsgemäß wird im zweiten Schritt das überkritische CO₂ bei dem gleichen Druck durch das Pflanzenmaterial geleitet wie bei dem ersten Schritt. Aus dem zweiten CO₂-Extrakt wird dann durch Absenken des Drucks ein carnosolsäurereicher Pflanzenextrakt abgeschieden. Aus dem ersten CO₂-Extrakt kann durch Absenken des Drucks ein ölreicher Pflanzenextrakt abgeschieden werden. Vorzugsweise werden bei dem Absenken des Drucks der Enddruck und die Endtemperatur so gewählt, dass das CO₂ vom überkritischen Zustand in den gasförmigen Zustand übergeht. Geeignete Abscheider für die Abscheidung des Pflanzenextrakts durch Absenken des Drucks sind dem Fachmann aus dem Stand der Technik zu Extraktionen mit überkritische CO₂ bekannt.

Das erfindungsgemäße Verfahren hat gegenüber der aus EP 454 097 bekannten Gewinnung eines carnosolsäurereichen Pflanzenextrakts durch zweistufiges Absenken des Drucks den Vorteil, dass ein carnosolsäurereicher Pflanzenextrakt mit einem geringeren Anteil an etherischen Ölen erhalten wird, der einen verringerten Geruch aufweist.

In einer bevorzugten Ausführungsform des erfindungsgemäßen Verfahrens wird der zweite CO₂-Extrakt vor dem Abscheiden des Pflanzenextrakts unter überkritischen Bedingungen durch ein festes Absorptionsmittel geleitet. Als Absorptionsmittel können dabei alle Absorptionsmittel verwendet werden, mit denen sich aus dem Pflanzenmaterial mitextrahierte Farbstoffe absorbieren lassen. Vorzugsweise werden als Absorptionsmittel Aktivkohle, Bleicherde (Fuller's earth), Kieselgur, Kieselgel oder Cellulose verwendet. Besonders bevorzugt wird als Absorptionsmittel eine Bleicherde verwendet, die aus einem calciumhaltigen Bentonit oder einem calciumhaltigen Montmorillonit besteht. Geeignete Bleicherden sind von der Firma Südchemie unter dem Handelsnamen Tonsil^{®} erhältlich. Das Absorptionsmittel kann in Pulverform oder vorzugsweise als Granulat eingesetzt werden. Mit dieser Ausführungsform können entfärbte carnosolsäurereiche Pflanzenextrakte hergestellt werden.

Der CO₂-Extrakt wird vorzugsweise unter den gleichen Bedingungen von Druck und Temperatur wie bei der Extraktion durch das festes Absorptionsmittel geleitet. Die Extraktion und das Durchleiten durch das Absorptionsmittel können in voneinander getrennten Apparaten oder im gleichen Apparat erfolgen.

Vorzugsweise wird auch der erste CO₂-Extrakt durch das feste Absorptionsmittel geleitet, bevor durch Absenken des Drucks der ölreiche Pflanzenextrakt abgeschieden wird, um auch den ölreichen Pflanzenextrakt entfärbt zu erhalten. Der erste CO₂-Extrakt und der zweite CO₂-Extrakt können dabei getrennt voneinander durch das gleiche oder durch unterschiedliche Absorptionsmittel geleitet werden. Vorzugsweise werden aber der erste CO₂-Extrakt und der zweite CO₂-Extrakt nacheinander durch das gleiche feste Absorptionsmittel geleitet.

In einer besonders bevorzugten Ausführungsform werden in einem Autoklav das carnosolsäurehaltige Pflanzenmaterial und das Absorptionsmaterial in Form von übereinanderliegenden Schichten angeordnet und das überkritische CO₂ wird erst durch die Schicht aus Pflanzenmaterial und danach durch die Schicht aus Absorptionsmaterial geleitet. Wenn das überkritische CO₂ dabei in Aufwärtsströmung durch die Schicht aus Pflanzenmaterial geleitet wird, wird dazu eine Schicht von Absorptionsmaterial oberhalb der Schicht aus Pflanzenmaterial angeordnet. Wenn das überkritische CO₂ in Abwärtsströmung durch die Schicht aus Pflanzenmaterial geleitet wird, wird eine Schicht von Absorptionsmaterial unterhalb der Schicht aus Pflanzenmaterial angeordnet. Diese Ausführungsform hat den Vorteil, dass in einer aus dem Stand der Technik bekannten Extraktionsanlage zur Extraktion mit überkritischem CO₂ ohne weitere Reinigungsvorrichtungen oder Reinigungsschritte ein entfärbter carnosolsäurereicher Pflanzenextrakt erhalten werden kann.

Das nachfolgende Beispiel illustriert die Erfindung, ohne jedoch den Gegenstand der Erfindung zu beschränken.

### Beispiel

Im dem Extraktionsbehälter eines Extraktionsautoklavs werden 1 kg auf einen Wassergehalt von 8 Gew.-% getrocknete und auf eine mittlere Partikelgröße von weniger als 1,5 mm gemahlene Rosmarinnadeln vorgelegt. Auf die Schüttung aus gemahlenen Rosmarinnadeln wird eine Schicht aus 100 g Bleicherde Tonsil^{®} Optimum 210 FF aufgebracht. Durch die Schüttung wird dann überkritisches CO₂ bei einem Druck von 280 bar und einer Temperatur von 65 °C in aufsteigender Strömung geleitet. Der erhaltene CO₂-Extrakt wird zunächst einem ersten Abscheider zugeführt, in dem bei einem Druck von 45 bar und einer Temperatur von 35 °C ein erster Pflanzenextrakt abgeschieden wird. Nachdem 30 kg überkritisches CO₂ durch die Schüttung geleitet waren, wurde der erhaltene CO₂-Extrakt einem zweiten Abscheider zugeführt, in dem bei einem Druck von 45 bar und einer Temperatur von 35 °C ein zweiter Pflanzenextrakt abgeschieden wurde. Insgesamt wurden 100 kg überkritisches CO₂ durch die Schüttung geleitet. Im ersten Abscheider wurden 77 g einer wässrigen Phase und 113 g eines orangefarbenen flüssigen Pflanzenextrakts erhalten, der 13 Gew.-% Carnosolsäure und 32 Gew.-% etherisches Öl enthielt. Im zweiten Abscheider wurden 48 g eines gelben viskosen Pflanzenextrakts erhalten, der 41 Gew.-% Carnosolsäure und weniger als 1 Gew.-% etherisches Öl enthielt.

## Patentansprüche

1. Verfahren zur Herstellung eines carnosolsäurereichen Pflanzenextrakts, umfassend Extraktion eines getrockneten und zerkleinerten carnosolsäurehaltigen Pflanzenmaterials mit überkritischem CO₂ bei einem Druck von mindestens 200 bar und einer Temperatur von höchstens 100 °C, **dadurch gekennzeichnet, dass** in einem ersten Schritt überkritisches CO₂ in einer Menge von 10 bis 50 kg CO₂ je kg Pflanzenmaterial durch das Pflanzenmaterial geleitet wird unter Erhalt eines ersten CO₂-Extrakts, in einem zweiten Schritt bei dem gleichen Druck weiteres überkritisches CO₂ durch das Pflanzenmaterial geleitet wird unter Erhalt eines zweiten CO₂-Extrakts und aus dem zweiten CO₂-Extrakt durch Absenken des Drucks ein carnosolsäurereicher Pflanzenextrakt abgeschieden wird.

2. Verfahren nach Anspruch 1,
**dadurch gekennzeichnet,**
**dass** das Pflanzenmaterial Blätter von Rosmarinum officinalis oder Salvia officinalis enthält.

3. Verfahren nach Anspruch 1 oder 2,
**dadurch gekennzeichnet,**
**dass** bei einem Druck von 280 bis 1000 bar, vorzugsweise 280 bis 420 bar, extrahiert wird.

4. Verfahren nach einem der Ansprüche 1 bis 3,
**dadurch gekennzeichnet,**
**dass** im zweiten Schritt 80 bis 250 kg CO₂ je kg Pflanzenmaterial durch das Pflanzenmaterial geleitet wird.

5. Verfahren nach einem der Ansprüche 1 bis 4,
**dadurch gekennzeichnet,**
**dass** aus dem ersten CO₂-Extrakt durch Absenken des Drucks ein ölreicher Pflanzenextrakt abgeschieden wird.

6. Verfahren nach einem der Ansprüche 1 bis 5,
**dadurch gekennzeichnet,**
**dass** der zweite CO₂-Extrakt vor dem Abscheiden des Pflanzenextrakts unter überkritischen Bedingungen durch ein festes Absorptionsmittel geleitet wird.

7. Verfahren nach Anspruch 6,
**dadurch gekennzeichnet,**
**dass** der erste CO₂-Extrakt und der zweite CO₂-Extrakt nacheinander durch das gleiche feste Absorptionsmittel geleitet werden.

8. Verfahren nach Anspruch 6 oder 7,
**dadurch gekennzeichnet,**
**dass** das Absorptionsmittel ausgewählt ist aus Aktivkohle, Bleicherde, Kieselgur, Kieselgel und Cellulose.

9. Verfahren nach einem der Ansprüche 6 bis 8,
**dadurch gekennzeichnet,**
**dass** eine Bleicherde ausgewählt aus calciumhaltigen Bentoniten und calciumhaltigen Montmorilloniten verwendet wird.

10. Verfahren nach einem der Ansprüche 6 bis 9,
**dadurch gekennzeichnet,**
**dass** das Absorptionsmittel in Form eines Granulats eingesetzt wird.

11. Verfahren nach einem der Ansprüche 6 bis 10,
**dadurch gekennzeichnet,**
**dass** das Pflanzenmaterial und das Absorptionsmaterial in einem Autoklav in Form von übereinanderliegenden Schichten angeordnet werden und das überkritische CO₂ erst durch die Schicht aus Pflanzenmaterial und danach durch die Schicht aus Absorptionsmaterial geleitet wird.

## Claims

1. Method for producing a carnosic acid-rich plant extract, comprising extraction of a dried and comminuted carnosic acid-containing plant material with supercritical CO₂ at a pressure of at least 200 bar and a temperature of at most 100°C, **characterized in that**, in a first step, supercritical CO₂ is passed through the plant material in an amount of 10 to 50 kg of CO₂ per kg of plant material, giving a first CO₂ extract, in a second step, at the same pressure, further supercritical CO₂ is passed through the plant material, giving a second CO₂ extract, and a carnosic acid-rich plant extract is precipitated from the second CO₂ extract by lowering the pressure.

2. Method according to Claim 1,
**characterized in that**
the plant material comprises leaves of Rosmarinum officinalis or Salvia officinalis.

3. Method according to Claim 1 or 2,
**characterized in that**
extraction is carried out at a pressure of 280 to 1000 bar, preferably 280 to 420 bar.

4. Method according to any one of Claims 1 to 3,
**characterized in that**
in the second step, 80 to 250 kg of CO₂ per kg of plant material is passed through the plant material.

5. Method according to any one of Claims 1 to 4,
**characterized in that**
an oil-rich plant extract is precipitated from the first CO₂ extract by lowering the pressure.

6. Method according to any one of Claims 1 to 5,
**characterized in that**
the second CO₂ extract is passed through a solid absorbent under supercritical conditions prior to precipitating the plant extract.

7. Method according to Claim 6,
**characterized in that**
the first CO₂ extract and the second CO₂ extract are passed in succession through the same solid absorbent.

8. Method according to Claim 6 or 7,
**characterized in that**
the absorbent is selected from activated carbon, bleaching earth, kieselguhr, silica gel and cellulose.

9. Method according to any one of Claims 6 to 8,
**characterized in that**
a bleaching earth selected from calcium-containing bentonites and calcium-containing montmorillonites is used.

10. Method according to any one of Claims 6 to 9,
**characterized in that**
the absorbent is used in the form of granules.

11. Method according to any one of Claims 6 to 10,
**characterized in that**
the plant material and the absorption material are arranged in an autoclave in the form of superimposed layers and the supercritical CO₂ is passed first through the layer of plant material and then through the layer of absorption material.

## Revendications

1. Procédé de fabrication d'un extrait végétal riche en acide carnosique, comprenant l'extraction d'un matériau végétal contenant de l'acide carnosique séché et broyé avec du CO₂ supercritique à une pression d'au moins 200 bar et une température d'au plus 100 °C, **caractérisé en ce que**, lors d'une première étape, du CO₂ supercritique est conduit en une quantité de 10 à 50 kg de CO₂ par kg de matériau végétal au travers du matériau végétal pour obtenir un premier extrait de CO₂, lors d'une seconde étape à la même pression, du CO₂ supercritique supplémentaire est conduit au travers du matériau végétal pour obtenir un second extrait de CO₂, et un extrait végétal riche en acide carnosique est séparé à partir du second extrait de CO₂ par réduction de la pression.

2. Procédé selon la revendication 1, **caractérisé en ce que** le matériau végétal contient des feuilles de Rosmarinum officinalis ou Salvia officinalis.

3. Procédé selon la revendication 1 ou 2, **caractérisé en ce que** l'extraction est réalisée à une pression de 280 à 1 000 bar, de préférence de 280 à 420 bar.

4. Procédé selon l'une quelconque des revendications 1 à 3, **caractérisé en ce que**, lors de la seconde étape, 80 à 250 kg de CO₂ par kg de matériau végétal sont conduits au travers du matériau végétal.

5. Procédé selon l'une quelconque des revendications 1 à 4, **caractérisé en ce qu'**un extrait végétal riche en huile est séparé à partir du premier extrait de CO₂ par réduction de la pression.

6. Procédé selon l'une quelconque des revendications 1 à 5, **caractérisé en ce que** le second extrait de CO₂ est conduit au travers d'un agent d'absorption solide dans des conditions supercritiques avant la séparation de l'extrait végétal.

7. Procédé selon la revendication 6, **caractérisé en ce que** le premier extrait de CO₂ et le second extrait de CO₂ sont conduits l'un après l'autre au travers du même agent d'absorption solide.

8. Procédé selon la revendication 6 ou 7, **caractérisé en ce que** l'agent d'absorption est choisi parmi le charbon actif, la terre activée, la diatomite, le gel de silice et la cellulose.

9. Procédé selon l'une quelconque des revendications 6 à 8, **caractérisé en ce qu'**une terre activée choisie parmi les bentonites contenant du calcium et les montmorillonites contenant du calcium est utilisée.

10. Procédé selon l'une quelconque des revendications 6 à 9, **caractérisé en ce que** l'agent d'absorption est utilisé sous la forme d'un granulat.

11. Procédé selon l'une quelconque des revendications 6 à 10, **caractérisé en ce que** le matériau végétal et le matériau d'absorption sont agencés dans un autoclave sous la forme de couches superposées et le CO₂ supercritique est tout d'abord conduit au travers de la couche de matériau végétal, puis au travers de la couche de matériau d'absorption.
